# NOUVEAU FASCICULE DE BREVET EUROPEEN

(11) **EP 2 953 564 B2**
(45) Date de publication et mention de la décision concernant l'opposition: **27.07.2022**
(45) Mention de la délivrance du brevet: 01.08.2018
(21) Numéro de dépôt: 14706889.4
(22) Date de dépôt: 05.02.2014
(51) Int. Cl.: A61B 17/88, A61B 17/70

(54) **ENSEMBLE D'IMPLANTATION COMPRENANT UN INSTRUMENT D'ENTRAÎNEMENT PRE-MONTÉ SUR UN IMPLANT OSSEUX**
IMPLANTATIONSANORDNUNG MIT EINEM AUF EINEM KNOCHENIMPLANTAT VORMONTIERTEN TREIBERELEMENT
IMPLANTATION ASSEMBLY COMPRISING A DRIVE INSTRUMENT PRE-FITTED ON A BONE IMPLANT

(30) Priorité: 05.02.2013 FR 1350989
(43) Date de publication de la demande: 16.12.2015
(73) Titulaire: Safe Orthopaedics, 95610 Eragny-sur-Oise (FR)
(72) Inventeur: PETIT, Dominique, F-62180 Verton (FR); DELAHAYE, Constant, F-95310 Saint Ouen L'Aumône (FR); FUENTES, Stéphane, 13012 Marseille (FR); MAESTRETTI, Gianluca, 1784 Wallenried (CH)
(74) Mandataire: IP Trust
(86) Numéro de dépôt international: PCT/FR2014/050214
(87) Numéro de publication internationale: WO 2014/122395

(56) Documents cités:
- FR-A1- 2 820 630
- FR-A1- 2 954 689
- US-A1- 2009 264 895
- US-A1- 2011 245 881
- US-A1- 2011 313 471
- US-A1- 2012 197 311

## Description

### DOMAINE TECHNIQUE DE L'INVENTION

L'invention concerne le domaine de l'ostéosynthèse, notamment rachidienne, et plus particulièrement celui de la pose d'implants osseux.

L'invention concerne plus particulièrement un ensemble d'implantation comprenant un élément d'ancrage osseux et un instrument d'entrainement de l'élément d'ancrage osseux, l'instrument étant destiné à entrainer par vissage l'élément d'ancrage osseux dans un trou préalablement foré ou non dans une structure osseuse.

### ETAT DE LA TECHNIQUE

A titre d'exemple, les éléments d'ancrage osseux pour une ostéosynthèse rachidienne comportent, de manière connue, une tige filetée pourvue, à l'une de ses extrémités, d'une tête apte à recevoir directement ou par l'intermédiaire d'un connecteur un élément de liaison.

Les éléments d'ancrage osseux sont destinés à être fixés sur les vertèbres concernées pour stabilisation et/ou ostéosynthèse. Pour ce faire, la tige filetée de l'élément d'ancrage osseux est positionnée dans un trou foré, préalablement ou non, dans une vertèbre. Une fois en place dans le trou, l'enfoncement de l'élément d'ancrage osseux dans la vertèbre est poursuivi à l'aide d'un instrument d'entrainement, du type tournevis, placé sur la tête de l'élément d'ancrage osseux. L'enfoncement de l'élément d'ancrage osseux est effectué jusqu'à atteindre un enfoncement suffisant pour assurer la fixation de l'élément d'ancrage osseux dans la vertèbre. Un ensemble d'implantation selon le préambule de la revendication 1 est connu de la demande de brevet américaine US 2012/0197311. Un élément d'ancrage osseux, pourvu d'une axe d'entraînement et disposé dans un emballage, est connu de la demande de brevet française 2 954 689 .

L'un des inconvénients rencontrés avec les instruments d'entrainement classiquement mis en œuvre est l'usure de leur extrémité d'accouplement avec la tête de l'élément d'ancrage osseux. Si cette usure est liée à l'utilisation répétée des instruments d'entrainement (appairage combiné au couple de serrage exercé par l'extrémité sur l'élément d'ancrage osseux), elle est également liée, voire accélérée, par des cycles récurrents de stérilisation après chaque intervention chirurgicale, au cours desquels les instruments subissent des actions agressives de solvants, de décontaminants, etc.. Lorsque les instruments d'entrainement sont usés, il s'ensuit une perte de rigidité de l'ensemble instrument d'entrainement/élément d'ancrage osseux, augmentant alors les risques de mauvais placement de l'élément d'ancrage osseux dans le corps vertébral.

Il n'est par ailleurs pas rare pour un chirurgien de rencontrer des difficultés d'appairage de l'instrument d'entrainement avec l'élément d'ancrage osseux. Ces difficultés sont d'autant plus importantes que l'élément d'ancrage osseux présente une tête mobile en rotation sur la tige filetée (vis multiaxiale). Or, de telles difficultés d'appairage peuvent avoir des conséquences lourdes en termes non seulement de durée d'une opération chirurgicale mais également de risques opératoires. Les temps chirurgicaux étant plus longs, il est alors en général nécessaire de recourir à des durées d'anesthésie plus longues. Afin de palier ces difficultés d'appairage d'un instrument d'entrainement avec un élément d'ancrage osseux, il est d'usage de prévoir une tolérance au niveau de la liaison instrument/élément d'ancrage. Le montage de l'instrument d'entrainement sur l'élément d'ancrage osseux est alors réalisé au détriment du maintien de l'élément d'ancrage osseux par l'instrument d'entrainement. Il est alors fréquent de « perdre » la liaison entre l'élément d'ancrage osseux et l'instrument d'entrainement, en particulier lors d'un effort de flexion sur l'instrument d'entrainement et donc sur l'élément d'ancrage osseux.

L'invention vise à remédier à ces problèmes en proposant un ensemble d'implantation supprimant tout problème d'usure des instruments d'entrainement et tout problème d'appairage entre un tel instrument et un élément d'ancrage osseux au cours d'une intervention chirurgicale.

L'invention a également pour objet de proposer un ensemble d'implantation fonctionnel permettant d'assurer, à chaque intervention chirurgicale, des instruments chirurgicaux dépourvus de toute usure et dont la stérilité est garantie.

L'invention a également pour objet de proposer un ensemble d'implantation autorisant une tolérance optimale de sorte à assurer le maintien de l'instrument d'entrainement sur l'élément d'ancrage osseux, notamment lorsqu'ils sont soumis à des efforts de flexion.

### OBJET DE L'INVENTION

A cet effet, l'invention concerne un ensemble d'implantation tel qu'énoncé par la revendication 1.

Par le terme « pré-monté », on entend un instrument d'entrainement dont l'axe d'entrainement est couplé de manière solidaire à l'élément d'ancrage osseux, et ce préalablement à l'implantation de l'élément d'ancrage osseux.

Ainsi, du fait d'un pré-montage d'un instrument d'entrainement à usage unique sur l'élément d'ancrage osseux, le problème d'usure de l'instrument d'entrainement ainsi que le problème d'appairage entre les deux pièces sont évités conjointement. De ce fait, une réduction de la tolérance est autorisée et le maintien rigide de l'élément d'ancrage osseux par l'instrument d'entrainement est assuré lorsque l'ensemble est soumis à des efforts de tension.

De même, le pré-montage de l'instrument d'entrainement sur l'élément d'ancrage osseux permet d'une part un gain de temps chirurgical du fait de la suppression de l'opération de mise en place de l'instrument d'entrainement sur l'élément d'ancrage osseux et d'autre part une diminution du risque opératoire, la durée d'anesthésie étant d'autant diminuée.

L'ensemble d'implantation comporte des moyens de retenue axiale de l'axe d'entrainement sur l'élément d'ancrage osseux.

Avantageusement, la tête est montée sur la tige filetée libre en rotation par rapport à la tige filetée.

Selon une configuration particulière, la tête présente une cavité longitudinale taraudée débouchant dans un canal transversal apte à recevoir un élément de liaison, l'axe d'entrainement dudit instrument présentant une extrémité coopérant avec le fond du canal transversal de la tête de l'élément d'ancrage osseux.

Selon une configuration particulièrement avantageuse, l'ensemble d'implantation comporte au moins un tube d'extension de l'élément d'ancrage osseux dimensionné pour recevoir en son sein l'axe d'entrainement, le tube d'extension présentant une extrémité d'accouplement avec la tête de l'élément d'ancrage osseux. De manière avantageuse, le tube d'extension est prévu pour que, lorsqu'il est couplé à la tête de l'élément d'ancrage osseux, il est agencé avec l'axe d'entrainement de façon à permettre le mouvement de rotation de l'axe d'entrainement autour de son axe longitudinal.

Selon une autre configuration avantageuse, ledit ensemble d'implantation comporte un tube de protection des tissus arrangé pour recevoir en son sein l'élément d'ancrage osseux.

Selon l'invention, l'élément d'ancrage osseux, sur lequel est pré-monté au moins l'instrument d'entrainement, est disposé dans un emballage scellé de conditionnement stérile.

Avantageusement, l'emballage de conditionnement stérile comporte en outre le tube d'extension et/ou le tube de protection.

Avantageusement, l'ensemble d'implantation est stérile.

Avantageusement, l'ensemble d'implantation est à usage unique.

Avantageusement, l'instrument d'entrainement forme un guide pour un instrument chirurgical présentant un corps tubulaire. Il permet ainsi de fixer à nouveau sur l'élément d'ancrage osseux un instrument de correction du type tube.

Avantageusement, l'axe d'entrainement est une barre de tournevis.

Avantageusement, l'instrument d'implantation est à usage unique.

Selon l'invention, la tige filetée comprend un alésage axial présentant une ouverture d'extrémité au niveau de la tête et au moins une lumière radiale communiquant avec l'alésage axial, et l'instrument d'entrainement comporte un axe d'entrainement pré-monté de manière amovible sur l'élément d'ancrage osseux, l'axe d'entrainement étant traversé longitudinalement par un canal de passage communiquant avec l'alésage axial de l'élément d'ancrage osseux.

Par le terme « communiquant », on entend un canal de passage agencé pour donner accès à l'alésage axial de l'élément d'ancrage osseux. Il peut s'agir d'une communication directe ou indirecte.

Comme indiqué précédemment, par le terme « pré-monté », on entend un instrument d'entrainement dont l'axe d'entrainement est couplé de manière solidaire à l'élément d'ancrage osseux, et ce préalablement à l'implantation de l'élément d'ancrage osseux.

L'ensemble d'implantation ainsi configuré permet d'assurer la distribution d'une substance de fixation, tel que du ciment, ou de tout autre produit, comme par exemple une substance de traitement osseux, dans la structure osseuse dans laquelle l'élément d'ancrage osseux est implanté, en supprimant tout risque de fuite de la substance distribuée en dehors de la structure osseuse lors de sa distribution.

En outre, cette configuration autorise la distribution d'une substance durant l'entrainement de l'élément d'ancrage osseux par l'instrument d'entrainement. Cela a pour avantage de ne pas limiter la distribution de la substance dans des zones localisées de la structure osseuse correspondant à l'emplacement de la lumière radiale de la tige filetée mais au contraire de la distribuer sur une hauteur plus importante. Lorsque la substance distribuée est une substance de fixation, il s'ensuit une amélioration de la fixation de l'élément d'ancrage osseux dans la structure osseuse.

En outre, la tige filetée peut comporter une pluralité de lumières radiales disposées par paire, chaque paire de lumières étant disposée à une même distance les unes des autres, les lumières radiales de chaque paire étant disposées diamétralement opposées. Selon une autre configuration avantageuse, la tige filetée comporte une pluralité de lumières radiales disposées à égale distance les unes des autres dans le sens axial et équidistantes les unes des autres de 120 degrés dans le sens radial.

Un procédé de préparation d'un ensemble d'implantation comprenant un élément d'ancrage comportant une tige filetée destinée à être implantée dans une structure osseuse et une tête comprend une étape de montage d'un tube d'extension sur la tête de l'élément d'ancrage osseux, le tube d'extension étant guidé le long d'un axe d'entrainement d'un instrument d'entrainement pré-monté sur l'élément d'ancrage osseux.

Une fois le tube d'extension en place, il peut être prévu de démonter l'instrument d'entrainement de l'élément d'ancrage osseux.

### BREVE DESCRIPTION DES FIGURES

D'autres objets et avantages de l'invention apparaîtront au cours de la description qui suit, faite en référence aux dessins annexés, dans lesquels :
- la figure 1 représente une vue schématique d'un ensemble d'implantation comprenant un instrument d'entrainement pré-monté sur un élément d'ancrage osseux ;
- la figure 4 représente une vue schématique d'un ensemble d'implantation selon un mode de réalisation de l'invention ;
- la figure 5 représente une vue en coupe longitudinale de l'ensemble d'implantation de la figure 4 selon l'axe V-V ;
- la figure 6 représente une vue de détail de l'axe d'entrainement de l'ensemble d'implantation de la figure 4 ;
- la figure 7 représente une vue d'un kit d'instruments comprenant l'ensemble d'implantation de la figure 1 et un tube d'extension ;
- la figure 8 illustre une vue d'un kit d'instruments comprenant l'ensemble d'implantation de la figure 1 et un tube de protection des tissus ;
- la figure 13 représente une vue schématique en coupe d'un ensemble d'implantation selon un deuxième mode de réalisation.

### DESCRIPTION DETAILLEE DES FIGURES

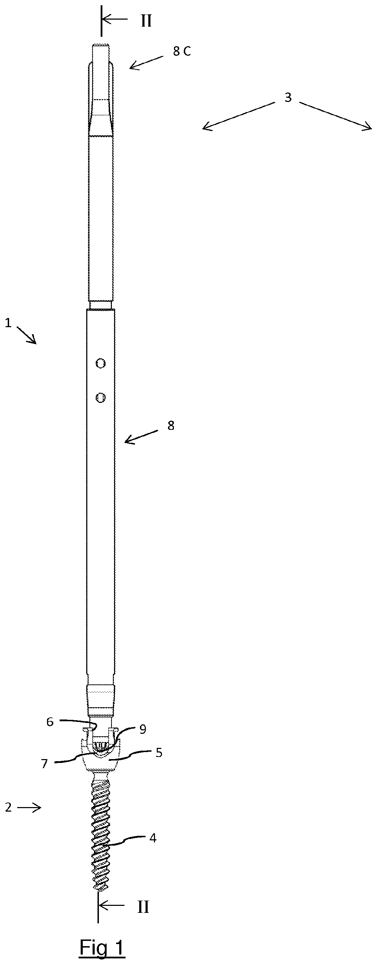

En relation avec la figure 1, il est décrit un ensemble d'implantation 1 pour ostéosynthèse selon un mode de réalisation, ledit ensemble d'implantation comprenant un élément d'ancrage osseux 2 sur lequel est pré-monté de manière amovible un instrument d'entrainement 3 de l'élément d'ancrage osseux 2.

Dans ce qui suit, l'élément d'ancrage osseux 2 pourra être également désigné par le terme de vis.

L'élément d'ancrage osseux 2 comporte une tige filetée 4 pourvue à l'une de ses extrémités 40 d'une tête 5 en forme de tulipe. La tête 5 présente ainsi une cavité longitudinale 6 taraudée débouchant dans un canal transversal 7. De manière classique en soi, le canal transversal 7 est destiné à recevoir un élément de liaison, par exemple une tige de liaison, la cavité longitudinale taraudée 6 étant destinée à recevoir un bouchon de blocage de l'élément de liaison sur la tête 5 de l'élément d'ancrage osseux 2.

Dans le mode de réalisation décrit, l'élément d'ancrage osseux 2 est une vis multiaxiale. Plus particulièrement, la tête 5 est montée libre en rotation sur la tige filetée 4. Pour ce faire, la tige filetée 4 comporte une extrémité 40 de forme sphérique logée en partie inférieure de la tête 5, dans un espace ménagé sous le canal transversal 7 et débouchant dans ce dernier. L'espace présente une ouverture de sortie pour le passage de la tige filetée 4 lors du montage de l'élément d'ancrage osseux dans la tête 5.

Avantageusement, une pièce formant berceau 9 est prévue au fond du canal transversal 7 de la tête 5 de vis, ladite pièce présentant un logement supérieur destiné à recevoir l'élément de liaison une fois l'instrument d'entrainement 3 retiré. La pièce formant berceau 9 présente en outre une face inférieure de forme complémentaire à celle sur laquelle elle est destinée à être placée. Dans le mode de réalisation illustré, la face inférieure présente une forme complémentaire à celle de l'extrémité 40 de la tige filetée. La pièce formant berceau 9 comporte en outre un trou traversant pour permettre le passage d'une portion de l'instrument d'entrainement 3.

Dans le mode de réalisation décrit, l'instrument d'entrainement 3 comporte un axe d'entrainement 8 présentant une extrémité 80 agencée pour coopérer avec le fond du canal transversal 7 de la tête 5 de la vis 2. On désignera par la suite cette extrémité 80 d' « extrémité de raccordement ou d'accouplement 80 ». Dans le mode de réalisation illustré, la tête 5 et la tige filetée 4 de l'élément d'ancrage osseux 2 sont agencées l'une par rapport à l'autre de manière à ce que la tige filetée 4 débouche dans le canal transversal 7. L'axe d'entrainement 8 est alors pré-monté directement sur l'extrémité 40 de la tige filetée débouchant dans le canal transversal 7. Comme représenté sur la figure 2, l'extrémité 40 de la tige filetée comporte une cavité 41 de forme complémentaire avec l'extrémité de raccordement 80 de l'axe d'entrainement 8.

Avantageusement, comme illustré sur la figure 1, la pièce de préhension et de manipulation 8A est pourvue d'une empreinte de réception 8C d'une poignée de préhension (poignée non représentée). La présence d'une poignée a pour avantage de faciliter la manipulation de l'axe d'entrainement 8.

Afin d'améliorer la retenue de l'axe d'entrainement 8 sur l'élément d'ancrage osseux 2, l'ensemble d'implantation 1 comporte avantageusement une pièce intermédiaire de maintien disposée entre la tête 5 de l'élément d'ancrage osseux 2 et l'axe d'entrainement 8 de l'instrument. La pièce intermédiaire de maintien est arrangée avec la tête 5 et l'axe d'entrainement 8 pour maintenir ce dernier dans l'alignement de la tige filetée 4 de l'élément d'ancrage osseux 2 sur lequel l'instrument d'entrainement 3 est pré-monté.

Avantageusement, la pièce intermédiaire de maintien est réalisée en un matériau souple, comme par exemple un élastomère. La pièce intermédiaire de maintien en élastomère présente avantageusement une section légèrement supérieure à la section de la cavité longitudinale 6 de la tête de vis 5. Ainsi, lors du montage de l'axe d'entrainement 8 sur l'élément d'ancrage osseux, la pièce intermédiaire formée de matériau souple se déforme. La pièce intermédiaire ainsi libérée permet de maintenir l'axe d'entrainement 8 couplé à l'élément d'ancrage osseux 2.

La pièce intermédiaire de maintien est dimensionnée pour recouvrir au moins la portion de la pièce de raccordement 8B s'étendant dans la cavité longitudinale 6 lorsque l'instrument d'entrainement 3 est pré-monté sur l'élément d'ancrage osseux 2. Dans le mode de réalisation décrit, la pièce intermédiaire de maintien présente avantageusement une longueur égale à la profondeur P de la cavité longitudinale auquel s'ajoute la longueur L de la portion de l'axe d'entrainement 8 s'étendant entre l'ouverture de la cavité longitudinale 6 de la tête de vis 6 et l'extrémité 90 de la pièce de préhension 8A. En prévoyant une telle longueur de la pièce intermédiaire, il suffit de glisser la pièce intermédiaire de maintien le long de l'axe d'entrainement 8 jusqu'à venir en butée sur la face inférieure de la pièce de préhension 8A pour assurer un positionnement correct de la pièce de maintien sur l'axe. Par positionnement correct, on entend qu'une portion de la pièce intermédiaire de maintien s'étend dans la cavité longitudinale 6. Le terme « inférieur » est donné en référence aux figures. Par ailleurs, en prévoyant une mise en butée sur la pièce de préhension 8A, tout glissement de la pièce intermédiaire de maintien lors du pré-montage de l'instrument d'entrainement 3 sur l'élément d'ancrage osseux 2 est empêché.

Avantageusement, la pièce intermédiaire de maintien est montée sur l'axe d'entrainement 8 de manière amovible.

Outre l'implantation de l'élément d'ancrage osseux 2 dans une vertèbre, l'axe d'entrainement 8 constitue un guide pour des instruments chirurgicaux présentant un corps tubulaire. En particulier, il peut être mis en oeuvre pour guider un tube servant luimême de guide pour l'élément de liaison.

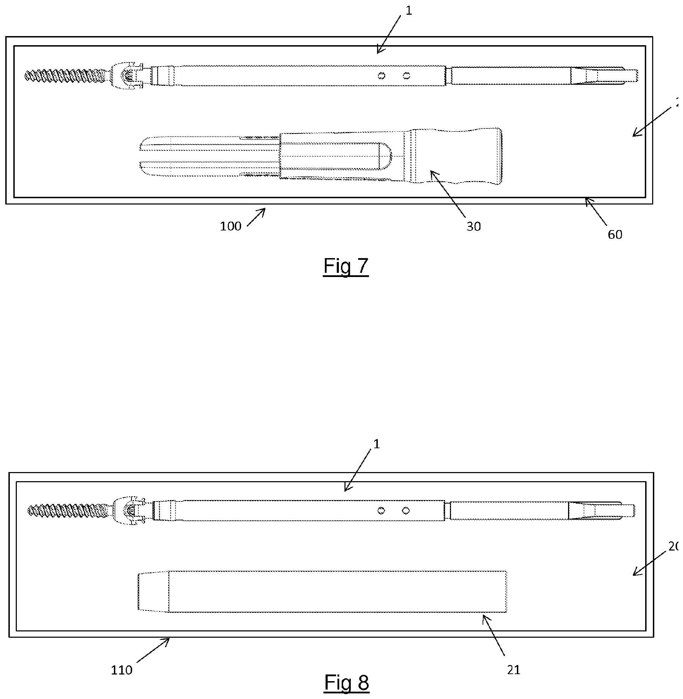

Selon un mode de réalisation particulier non illustré, il peut être prévu un ensemble d'implantation comprenant un tube d'extension de l'élément d'ancrage osseux, ledit tube étant pré-monté sur la tête 5 de vis et traversé en son sein par l'axe d'entrainement 8. Avantageusement, le tube d'extension présente une section interne permettant le mouvement de rotation axiale de l'axe d'entrainement 8. Un tel tube est représenté non monté sur la figure 7 (référence 30).

Comme l'illustre la figure 7 , l'élément d'ancrage osseux 2 pré-monté de l'instrument d'entrainement 3 selon l'invention est disposé dans un emballage de conditionnement 20 stérile. Dans ce même emballage, il est prévu également un tube d'extension 30 de l'élément d'ancrage osseux apte à être monté sur l'élément d'ancrage osseux 2. De manière avantageuse, le tube de d'extension 30 est agencé pour que, lorsqu'il est couplé à la tête 5 de l'élément d'ancrage osseux 2, il est traversé par l'axe d'entrainement 8 et est agencé avec celui-ci de façon à permettre le mouvement de rotation dudit axe d'entrainement 8 autour de son axe longitudinal (axe longitudinal de l'axe d'entrainement 8). L'ensemble d'implantation et le tube d'extension 30 ainsi disposés dans un emballage de conditionnement stérile 20 forment un kit 100. Le kit 100 illustré comporte un ensemble d'implantation 1 et un tube d'extension 30. Il est bien entendu évident que le kit ne se limite pas à ces deux instruments et qu'il peut comporter plusieurs ensembles d'implantation comme plusieurs tubes de montage ou comme tout autre type d'instruments à usage unique nécessaire à une intervention chirurgicale. Selon une autre variante de réalisation non représentée, il peut être prévu de réaliser un kit comprenant un ensemble d'implantation tel que décrit précédemment sur lequel le tube d'extension est également pré-monté. Le kit pourra comprendre, comme celui décrit précédemment, tout type d'instruments à usage unique nécessaire à une intervention chirurgicale.

Selon une variante de réalisation illustrée sur la figure 8, il peut être prévu de réaliser un kit 110 comprenant un ensemble d'implantation 1 tel que décrit précédemment et un tube de protection ou de dilatation 21 des tissus ou un ensemble de tubes de protection/dilation emboités les uns dans les autres, et disposés dans un emballage stérile 20. La figure 8 illustre un ensemble d'implantation 1 et un tube ou ensemble de tubes de protection/dilatation 21 non pré-monté(s). Il peut être prévu bien entendu un kit dans lequel le ou les tubes de protection/dilation des tissus est(sont) pré-monté(s) sur l'élément d'ancrage osseux, l'axe d'entrainement traversant le(s)dit(s) tube(s).

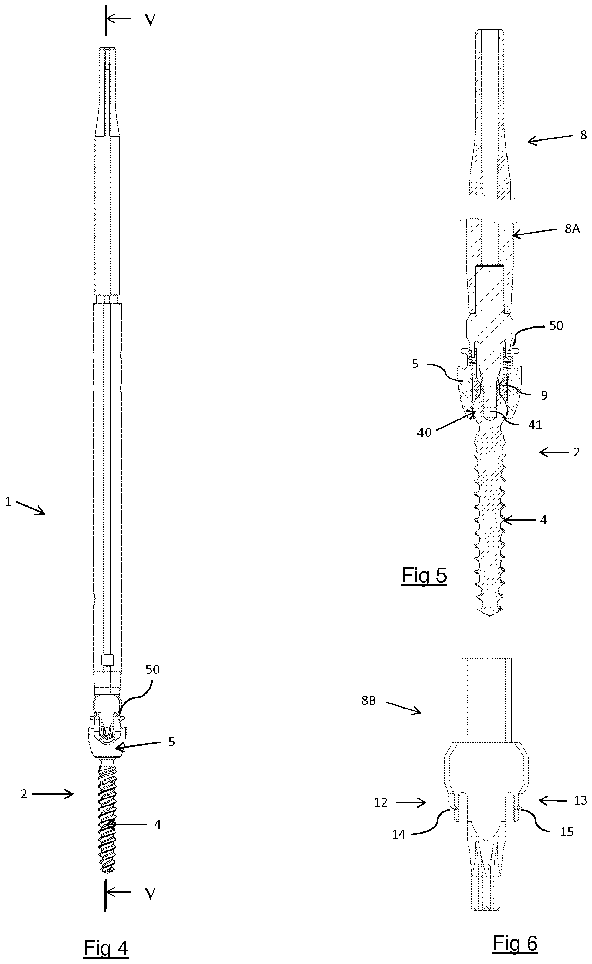

Les figures 4 à 6 illustrent un ensemble d'implantation 1 selon un autre mode de réalisation. Dans ce mode de réalisation, les moyens de retenue de l'axe d'entrainement 8 sur l'élément d'ancrage osseux 2, formés dans le mode de réalisation précédemment décrit par la pièce intermédiaire de maintien, sont ménagés directement sur l'axe d'entrainement 8.

Plus particulièrement, la pièce de raccordement 8B comporte deux pattes élastiques 12, 13 s'étendant de part et d'autre de son corps suivant une direction sensiblement longitudinale. Lesdites pattes 12, 13 sont arrangées de manière à venir s'enchâsser dans la cavité longitudinale 6 de la tête de vis 5 lorsque l'instrument d'entrainement 3 est pré-monté sur l'élément d'ancrage osseux 2. Afin d'assurer une retenue suffisante de l'axe d'entrainement 3 dans la tête 5 de vis, la pièce de raccordement 8B présente, au niveau de la portion d'axe délimitée par les pattes latérales 12, 13, une section externe légèrement supérieure à la section interne de la cavité longitudinale 6. Ainsi, lors du pré-montage de l'axe d'entrainement 8 sur la tête 5 de vis 2, les pattes élastiques 12, 13 sont rapprochées l'une de l'autre pour permettre l'insertion de la portion d'axe portant les pattes. Une fois relâchées, les pattes exercent une pression sur la surface interne de la cavité longitudinale 6 de la tête de vis 5, coinçant ainsi l'axe d'entrainement 3 dans la tête 2 de vis.

Avantageusement, les pattes flexibles 12, 13 comportent respectivement un épaulement externe 14, 15, chaque épaulement étant destiné à venir en butée sur la face supérieure 50 de la tête 5 de vis. Par l'expression « épaulement externe », on entend un épaulement s'étendant dans la direction opposée à la cavité longitudinale 6 lorsque l'instrument d'entrainement 3 est pré-monté sur l'élément d'ancrage osseux 2. Le terme « supérieur » est donné en référence aux figures.

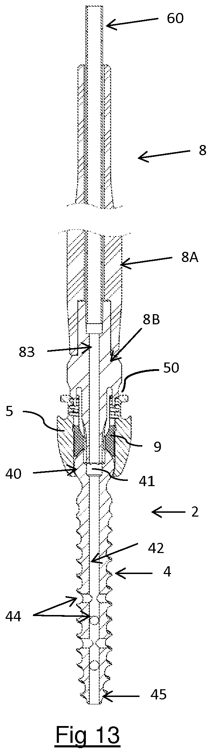

Dans le mode de réalisation illustré sur la figure 5, la pièce de raccordement 8B est représentée « pleine ». Selon un mode de réalisation avantageux, il peut être prévu que la pièce de raccordement 8B présente un canal longitudinal 83 comme la pièce de raccordement 8B représentée sur la figure 2. La pièce de préhension et de manipulation 8A étant elle même tubulaire, cela permet, lorsque l'instrument d'entrainement 3 est mis en oeuvre avec un élément d'ancrage osseux adapté (élément canulé avec fenêtres), d'injecter du ciment ou autre produit. Une telle configuration est illustrée sur la figure 13.

En complément de ces caractéristiques, l'élément d'ancrage osseux 2 comprend un alésage axial 42 s'étendant à partir de l'extrémité opposée à l'extrémité d'ancrage 45 (extrémité sphérique 40 pour le mode de réalisation illustré sur la figure 13). Il comprend en outre des lumières radiales 44 communiquant avec l'alésage axial 42 (seulement six sont représentées). Dans le mode de réalisation décrit, l'alésage axial 42 présente une ouverture d'extrémité au niveau de l'extrémité 40 de la tige filetée débouchant dans la cavité 41. L'alésage axial ne présente pas en revanche d'ouverture à l'extrémité opposée à l'extrémité 40, dans le sens axial. Dans l'exemple illustré l'élément d'ancrage osseux comprend huit lumières radiales (seulement six étant représentées). Il est bien entendu évident que le nombre de lumières radiales peut varier sans sortir du cadre de l'invention.

Selon une configuration particulière, les lumières radiales 44 sont disposées par paire. Les lumières radiales de chaque paire sont disposées diamétralement opposées, tandis que chaque paire est disposée à une distance déterminée les unes des autres. Avantageusement, les paires de lumières sont disposées à distance égale les unes des autres. Selon une autre configuration non représentée, les lumières sont disposées à égale distance les unes des autres dans le sens axial et équidistantes les unes des autres de 120 degrés dans le sens radial. Il est bien entendu évident que l'invention ne se limite pas à ces configurations, le nombre et l'emplacement des lumières pouvant varier sans sortir du cadre de l'invention.

Par ailleurs, et comme indiqué précédemment, l'instrument d'entrainement est canulé. Plus particulièrement, l'axe d'entrainement 8 est agencé pour présenter un canal de passage s'étendant sur toute la longueur dudit axe 8. Le canal est agencé de manière à communiquer, directement ou indirectement, dans l'alésage axial de l'élément d'ancrage osseux. Par communication directe, on désigne un canal de passage débouchant directement dans l'alésage axial de l'élément d'ancrage osseux; par communication indirecte, on entend un canal de passage communiquant avec l'alésage axial de l'élément d'ange osseux par l'intermédiaire d'une cavité : l'extrémité de raccordement 80 n'est pas enchâssée jusqu'au fond de la cavité 41 ménagée à l'extrémité 40 sphérique de l'élément d'ancrage osseux. De ce fait, le canal de passage débouche dans une portion de la cavité 41 qui elle-même communique avec l'alésage axial 42.

Avantageusement, l'injection de ciment ou autre substance est réalisée à l'aide d'une canule 60 insérée dans le canal de passage de l'axe d'entrainement. La figure 13 montre une vue partielle de la canule 60 montée dans le canal. Il est bien entendu évident qu'il s'agit d'un mode de réalisation particulier d'injection de substances. Le recours à une canule n'est en effet pas indispensable, le ciment et les autres substances pouvant être injectés directement dans le canal de passage.

Comme dans les modes de réalisation précédemment décrits, l'ensemble d'implantation illustré sur la figure 13 est disposé dans un emballage de conditionnement 20 stérile. Dans ce même emballage, il pourra être prévu également un ou plusieurs tube(s) d'extension de l'élément d'ancrage osseux apte à être monté sur l'élément d'ancrage osseux, un ou plusieurs tube(s) de protection ou de dilatation des tissus ou tout autre type d'instruments à usage unique nécessaire à une intervention chirurgicale de sorte à former un kit.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Ensemble d'implantation (1) comprenant un élément d'ancrage osseux (2) comportant une tige filetée (4) pourvue à l'une de ses extrémités d'une tête (5), la tige filetée (4) comprenant un alésage axial (42) présentant une ouverture d'extrémité au niveau de la tête (5) et au moins une lumière radiale (44) communiquant avec l'alésage axial (42), l'élément d'ancrage osseux (2) étant pourvu solidairement d'un axe amovible, traversé longitudinalement par un canal de passage communiquant avec l'alésage axial (42) de l'élément d'ancrage osseux pour l'injection de substances, ledit axe constituant un axe d'entrainement (8) d'un instrument d'entrainement (3) de l'élément d'ancrage osseux,
**caractérisé en ce que**
• ledit axe d'entrainement (8) comporte deux pattes flexibles (12, 13) s'étendant longitudinalement, lesdites pattes (12, 13) formant des moyens de retenue axiale dudit axe d'entrainement (8) sur ledit élément d'ancrage osseux (2)
• et **en ce que** ledit élément d'ancrage osseux (2) pourvu dudit axe est disposé dans un emballage scellé de conditionnement stérile (20).

## Patentansprüche

1. Implantationssatz (1) bestehend aus einem Knochenverankerungselement (2) mit Gewindestab (4), der an einem Ende mit einem Kopf () versehen ist, wobei der Gewindestab (4) eine axiale Bohrung (43) mit einer Endöffnung am Kopf (5) aufweist und mindestens eine radiale Öffnung (44), die mit der axialen Bohrung (42) in Verbindung steht, wobei das Knochenverankerungselement (2) mit einer abnehmbaren Achse verbunden ist, durch welche in Längsrichtung ein Durchführungskanal führt, der mit der axialen Bohrung (42) des Knochenverankerungselements zwecks Einspritzung einer Substanz in Verbindung steht, wobei besagte Achse die Antriebsachse (8) eines Antriebsinstruments (3) für ein Knochenverankerungselement ist,
**dadurch gekennzeichnet**
• die Antriebsachse (8) zwei sich längs erstreckende elastische Laschen (12, 13) aufweist, wobei besagte die Laschen (12, 13) axiale Mittel zum Halten der Antriebsachse (8) auf dem Knochenverankerungselement (2) umfasst
• und dass sich das Knochenverankerungselement (2) mit besagter Achse in einer sterilen versiegelten Verpackung (20) befindet.

## Claims

1. An implantation assembly (1) comprising a bone-anchoring element (2) having a threaded rod (4), one end of which is provided with a head (5), with said threaded rod (4) comprising an axial bore (42) having an end opening at the head (5) and at least one radial slot (44) which communicates with the axial bore (42), with the bone-anchoring element (2) being integrally provided with a removable axis, through which a flow path longitudinally goes, which communicates with the axial bore (42) of the bone-anchoring element for injecting substances, with said axis being a drive shaft (8) of a drive instrument (3) for the bone-anchoring element (2),
**characterized in that**
• the drive shaft (8) comprises two flexible lugs (12, 13) which extend longitudinally, with said lugs (12, 13) forming means for axially retaining said shaft (8) on said bone-anchoring element (2)
• and **in that** the bone-anchoring element (2) provided with said shaft is placed in a sealed sterile packaging (20).
